# EUROPEAN PATENT APPLICATION

(11) **EP 4 534 117 A1**
(43) Date of publication of application: **09.04.2025**
(21) Application number: 23306699.2
(22) Date of filing: 03.10.2023
(51) Int. Cl.: A61L 27/36, C12N 5/071

(54) **TRACHEAL DECELLULARIZATION TISSUE ENGINEERING PROTOCOL FOR CIRCUMFERENTIAL TRACHEAL REPLACEMENT**

(71) Applicant: Assistance Publique - Hôpitaux de Paris, 75012 Paris (FR)
(72) Inventor: THIERRY, Briac, 75013 PARIS (FR); ARAKELIAN, Lousineh, 75020 PARIS (FR); LARGHERO, Jérôme, 92100 BOULOGNE-BILLANCOURT (FR); CAPUTO, Valentino, 92240 MALAKOFF (FR)
(74) Representative: Novagraaf Technologies

(57) **Abstract**

The present invention concerns a new tracheal decellularization tissue engineering protocol for circumferential tracheal replacement, to develop a clinical-grade partially tracheal decellularized matrix.

## Description

### Technical field of the invention

The present invention concerns a new tracheal decellularization tissue engineering protocol for circumferential tracheal replacement, to develop a clinical-grade partially tracheal decellularized matrix.

The present invention finds its applications mainly in clinics, in particular in paediatrics.

In the description below, references in square brackets ([ ]) refer to the list of references at the end of the text.

### State of the art

Tracheal defects of less than 50% in adults and less than 30% in children can be reconstructed using an end-to-end anastomosis. Beyond this length, circumferential tracheal replacement of the resected area is necessary (Grillo, 1973) [1] which remains a surgical challenge because of the need of a tracheal substitute, for which there are currently no standards.

The ideal characteristics of a replacement tracheal substitute are: longitudinal flexibility movements of the neck and breathing, lateral rigidity and preservation of the diameter, passage of air under all circumstances (external stresses or constraints), reliable and reproducible acquisition technique, sterility and biocompatibility with low immunogenicity. A continuous wall covered by respiratory epithelium for mucus clearance would be an advantage. Particularly in paediatrics, the substitute must also be small in size; it must retain its biomechanical properties so that it can be implanted without the need for internal stents; and it must retain growth potential.

Numerous surgical techniques have been proposed over the years with mixed results. Autologous surgical reconstructions are technically extremely complex (Fabre *et al.,* 2013; Kolb *et al.,* 2018; Kubo *et al.,* 2018; Thomet *et al.,* 2018) [2-5]. Thyrotracheal complex allotransplantations are equally challenging due to the complexity of direct vascular restoration and long-term immunosuppressive therapy (Parshin *et al.,* 2018; Genden *et al.,* 2021) [6,7]. Synthetic products are not currently suitable for transplantation (Greaney and Niklason, 2021) [8]. 3D-printed bio-mimetic scaffolds have proven limited biointegration, with limited colonization by patient cells, limited neovascularization and quick degradation (Kaye *et al.,* 2019; Park *et al.,* 2019) [9,10]. Aortic allografts show satisfactory biointegration results but aortic malacia requires long-term stenting (Wurtz *et al.,* 2006, 2010; Martinod *et al.,* 2018) [11-13].

In this context, tissue-engineered decellularization of the trachea is an interesting area of research. This technique makes biological tissues less immunogenic by removing cells, antigens and DNA, thus providing enhanced biocompatibility with a low risk of rejection, while preserving the chemical and biomechanical properties of the extracellular matrix to guarantee bio-integration capacity, limit the risk of cartilage disease and avoid prolonged endoluminal stenting. Numerous complete decellularization protocols, removing all immunogenic cellular material, have been studied. One of the initial stumbling challenges was the malacia induced by full decellularization, carried out by long and aggressive treatment to remove mucosal cells and chondrocytes. Initial clinical applications have shown poor results (Elliott *et al.,* 2012, 2017; Culme-Seymour *et al.,* 2016; Parshin *et al.,* 2018) [14-16, 6].

Since epithelial cells, which express MHC class II, are the most immunogenic, while chondrocytes have a low immunogenicity (Liu *et al.,* 2000b, 2000a, 2001; Delaere *et al.,* 2010; Parshin *et al.,* 2018) [17-20, 6], several teams have demonstrated, in mouse and porcine tracheal models, the benefits of partial decellularization, which removes only cells from the mucosa, but preserves chondrocytes and the cartilage. This trade-off tissue engineering technique makes the matrix biocompatible without rendering it malacic (Aoki *et al.,* 2019; Liu *et al.,* 2021) [21,22]. However, previously published protocols were mainly based on small animal models whose tracheas are difficult to compare with those of humans. In addition, those studying pig tracheas showed that cells remained in the submucosa at the end of their protocol.

### Description of the invention

The inventors developed a new partial decellularization tissue engineering protocol, using a porcine model, to develop a clinical-grade partially decellularized tracheal (PDT) matrix and validate the biointegration *in vivo.* Depending on the pathology, decellularized trachea could be used as a replacement or external reinforcement.

The detergent based treatment for decellularization is longer than Aoki's protocol (Aoki et al., 2019) [21] but removes all the cells from the inner lining of the trachea, and is shorter than Lu and Liu's protocols (Y. Liu et al., 2002; Lu et al., 2018) [23,24], to avoid damaging the extracellular matrix. The technique can also include a storage phase in a tissue bank. This long-term storage at -80°C or -196°C is widely used for biological tissues, and can facilitate clinical logistics and allow emergency surgery in a clinical setting.

The choice of the porcine model was based on the principles of translational research: the results of the animal model study had to be usable for human inferences. Small animal models (mouse, rat, guinea pig) were thus ruled out from the outset, and the pig was chosen because its trachea is similar in size to that of humans, and its immune system is well-known and studied. The immunological results in this organ transplant study can also be used as a basis for extrapolation to humans.

After a thorough characterization of the final product: cell and DNA removal, sterility, toxicity, detergent quantification, biomechanical properties evaluation and in vivo study, the results showed that the final matrix was cell-free in the mucosa and the submucosa, while the less immunogenic cartilage was intact, maintaining the tracheal biomechanical properties and caliber. When implanted *in vivo* in a muscle for about one month, no systemic inflammation was observed, and the partially decellularized tracheal matrix showed excellent biointegration and vascularization, with no toxicity.

An object of the present invention is therefore a partial tracheal decellularization tissue engineering method comprising the following steps:
a) partial decellularisation of a trachea previously sampled from a mammal with a detergent;
b) rinsing the partially decellularized trachea recovered from step a) at least once with physiological saline solution to remove the detergent;
c) contacting the partially decellularized trachea recovered from step b) with active charcoal (cartridge) to adsorb residual detergent;
d) contacting the partially decellularized trachea recovered from step c) with a deoxyribonuclease (*e.g*. dornase alfa or pulmozyme^{®}) to remove residual DNA;
e) rinsing the partially decellularized trachea recovered from step d) at least once with physiological saline solution to remove the deoxyribonuclease;
f) optionally, cryopreservation of the partially decellularized trachea recovered from step e).

According to a particular embodiment of the present invention, the sampled trachea of step a) is defatted and decontamined before decellularization by means well-known in the art. For example, the sampled trachea is surgically defatted. For example, the sampled trachea is contacted with antibiotic(s) and/or antimycotic(s), in particular overnight, at room temperature, under stirring.

According to a particular embodiment of the present invention, the mammal from which the trachea was previously sampled is selected from a mammal of at least about 3kg, for example from pig, human, dog, sheep, goat, cow, non-human genetically modified mammals, and humanized mammals.

According to a particular embodiment of the present invention, step a) is carried out between 12 to 36 hours, preferably during about 24 hours, at room temperature.

According to a particular embodiment of the present invention, the detergent of step a) is selected from Sodium Dodecyl Sulfate (SDS), Triton X-100, Tween-20, sodium deoxycholate. For example, the detergent is SDS 1%.

According to a particular embodiment of the present invention, step c) is carried out during 96 hours, at room temperature.

According to a particular embodiment of the present invention, step c) and e) are carried out three times with stirring.

According to a particular embodiment of the present invention, the partially decellularized trachea in step f) is placed at -20°C in RPMI medium during 12 hours then stocked at -80°C. In particular, the RPMI medium contain DMSO 10% and albumin 0.8%.

Another object of the present invention is also the partially decellularized trachea obtained by a method of the present invention, for use as a medicament. Preferably, the medicament is a paediatric medicament.

Another object of the present invention is also the partially decellularized trachea obtained by a method of the present invention, for use in the prevention or treatment of acquired or congenital stenosis of the trachea (*e.g*. congenital tracheal stenosis), large tracheoesphageal fistula, tracheal agenesis, tracheal tumor, laryngotrachealoesphageal cleft, tracheomalacia.

### Advantages of the present invention :

- simplification of the partial decellularisation procedure with a single detergent treatment step.
- preferential use of products that are already approved for food industry and medical use (SDS, pulmozyme^{®}, active charcoal cartridge) as much as possible.
- high efficiency of the partial decellularization procedure to eliminate cells in the mucosa and submucosa, while preserving the cartilage and properties thereof.
- absence of DNA in the mucosa and submucosa.
- PDT sterile from the first decontamination step, and sterility maintained up to and after cryopreservation.
- absence of cytotoxicity *in vitro* on Balb/3T3 and bronchial epithelial cells and *in vivo* on recipient muscle.
- no residual detergent (detoxification phase).
- preservation of biomechanical properties.
- first protocol that is easily transferable to a clinical-grade production entity.
- the only large animal model trachea to show complete decellularisation of the tunica interna of the trachea.
- the only partially decellularised trachea for which the clinical grade was tested: sterility, absence of cytotoxicity.

### Brief description of the figures

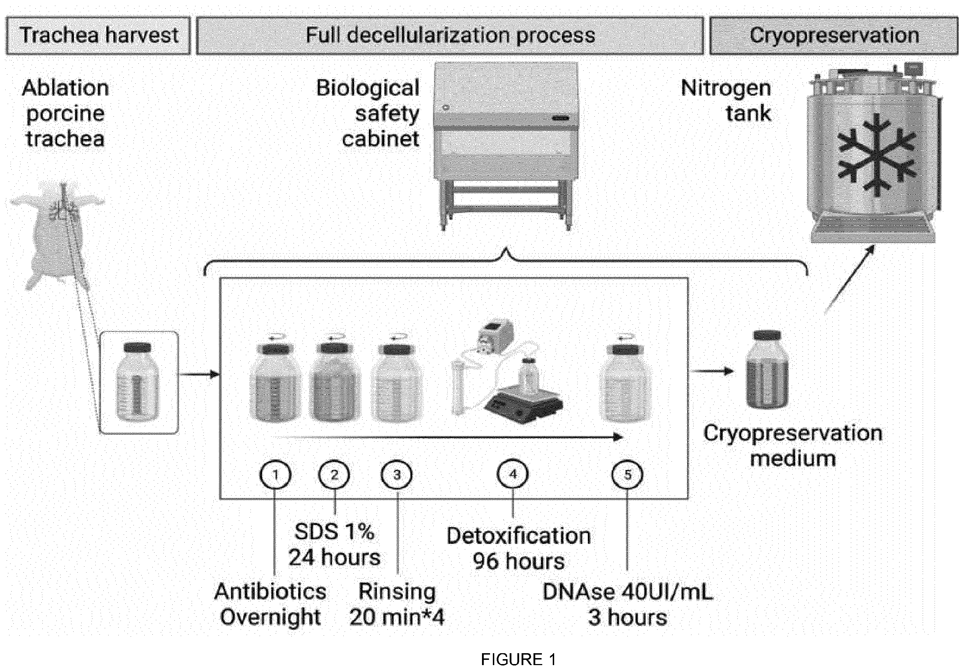

Figure 1 shows a diagram of decellularization steps from porcine tracheal harvest to cryopreservation. The partial decellularization process is represented. The five steps of decellularization were: decontamination, SDS treatment, rinsing, detoxification and DNAse treatment. Cryopreservation enabled long-term storage. All steps were performed in a bottle, under sterile conditions in a laminar flow hood.

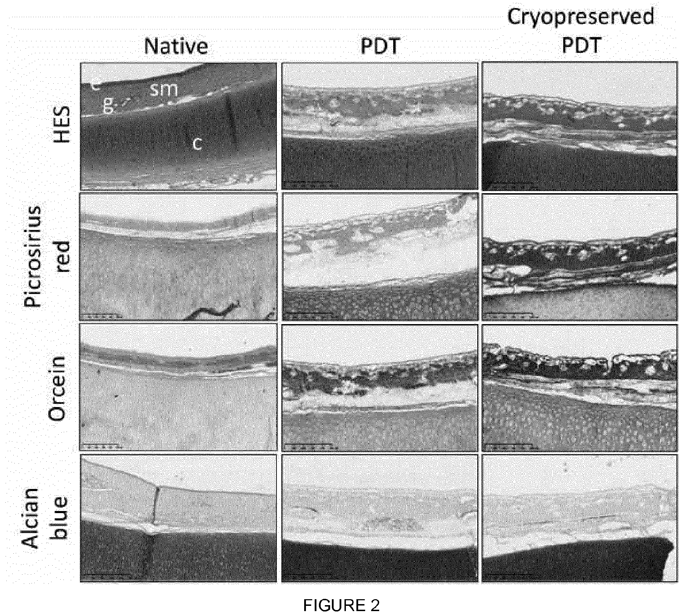

Figure 2 shows the histological appearance of a porcine tracheas native (left), partially decellularized (PDT, middle) or partially decellularized and cryopreserved (PDT cryopreserved, right) using hematoxylin-eosin-saffron (HES) for the general structure, Picrosirius red (PSR) for collagen, orcein for elastic fibers and Alcian Blue for glycosaminoglycan staining. Epithelium (e), sub-mucosa (sm), sub-mucosal gland (g) and cartilage (c) are shown in HES native trachea. Scale bars = 500 µm. PDT: Partially decellularized trachea. Porcine donor trachea is efficiently partially decellularized.

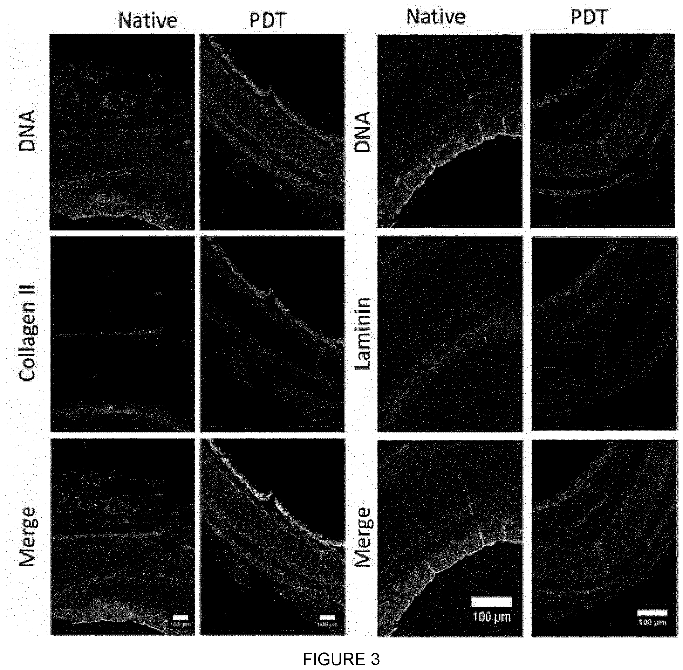

Figure 3 shows immunofluorescence staining of native trachea and PDT using antibodies against collagen II and laminin. Cell nuclei (DNA) were stained with DAPI. The lower images were the merged images of collagen II and DNA, and Laminin and DNA respectively. Scale bar = 100 µm. PDT: partially decellularized trachea.

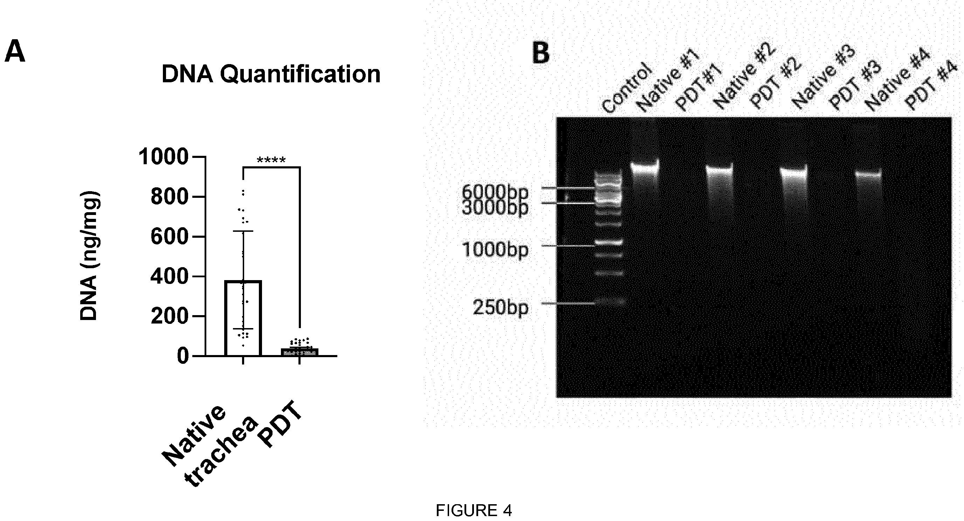

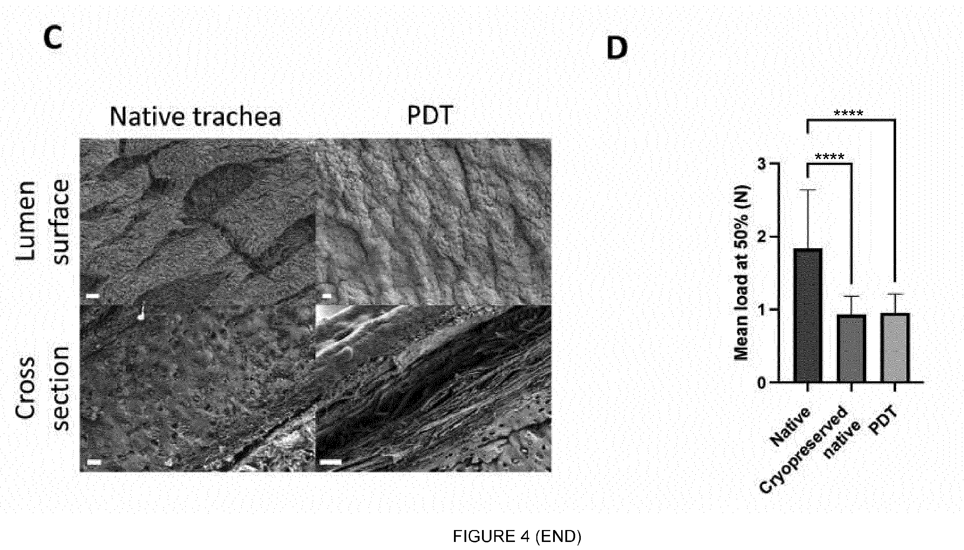

Figure 4 shows (**A**) DNA amount (ng/mg) in native trachea and PDT. Thirty donor matched tracheas were assessed and data were analyzed using a Mann-Whitney test (**** = p < 0.0001). (**B**) DNA electrophoresis on agarose gel. 30-mg mucosal sample of four matched tracheas were assessed, native and after decellularization. (**C**) Scanning electron microscopy (SEM) images of lumen surface and cross-section of porcine native trachea and PDT samples. Upper panel scale bar = 10 µm, lower panel scale = 20 µm. (**D**) Radial compression of native trachea, cryopreserved trachea and PDT. PDT: partially decellularized trachea (**** = p < 0.0001).

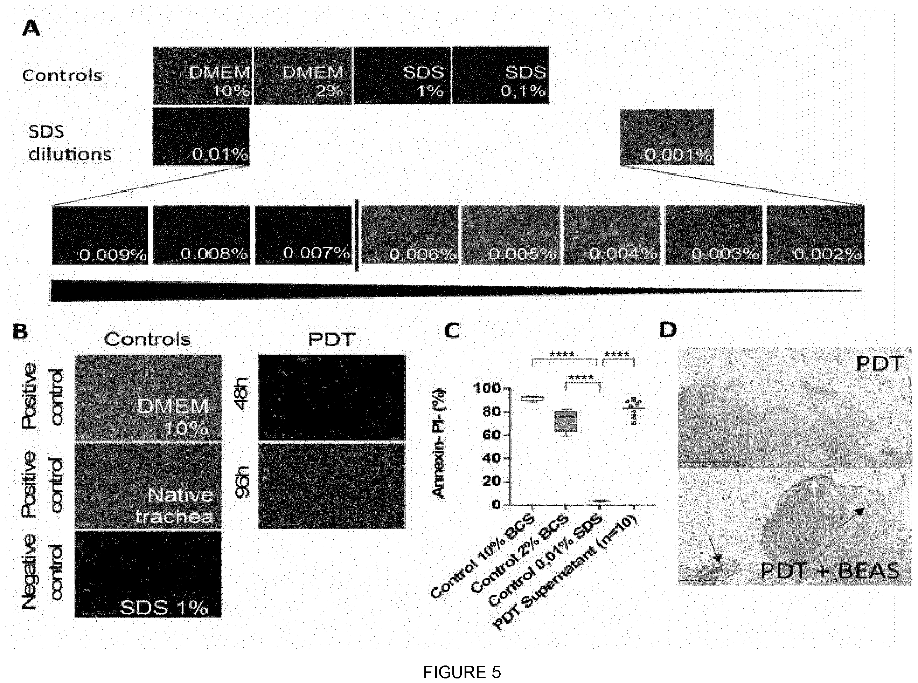

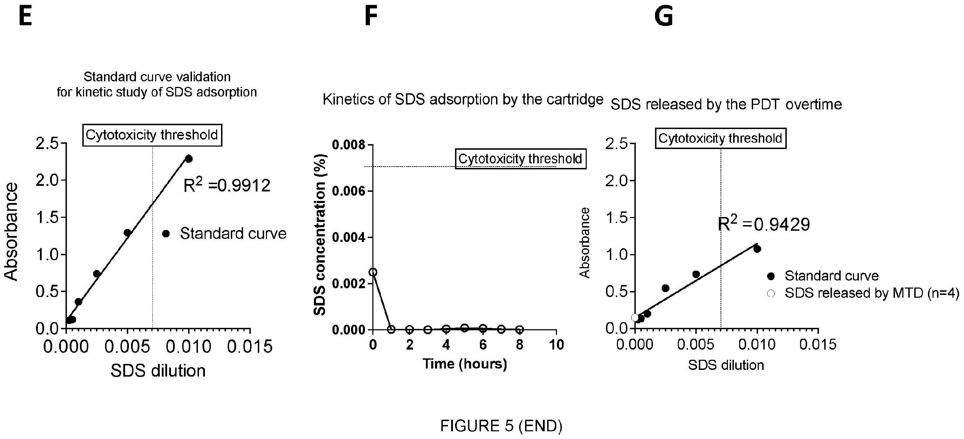

Figure 5 shows *In-vitro* cytotoxicity study. (**A**) Viability of BALB/3T3 was assessed with decreasing concentrations of Sodium dodecylsulfate (SDS), ranging from 0.01% to 0.001%. Positive and negative controls cell culture medium were Dulbecco's Modified Eagle Medium (DMEM) 10%, 2% and SDS 1%. Fluorescence was assessed after a 48-hours culture with various SDS concentrations. Green and red fluorescence were obtained with LIVE/DEAD^{®} viability/cytotoxicity kit. Green represents viability and red represents cell death. The viability threshold is represented as a vertical red line. Experiments were performed in duplicate. (**B**) Viability of BALB/3T3 was tested in the presence of partially decellularized tracheas (PDT) supernatants collected at two different times during detoxification (48 and 96 hours). Positive and negative controls cell culture medium were Dulbecco's Modified Eagle Medium (DMEM) 10%, native trachea supernatant after decontamination and SDS 1%. Fluorescence was assessed after a 48-hours culture with the supernatant taken from the detoxification in progress. Green and red fluorescence were obtained with LIVE/DEAD^{®} viability/cytotoxicity kit. After 48 hours of detoxification with the charcoal cartridge, supernatant was still cytotoxic. At 96 hours, cells were viable. (n=15). (C) Flow cytometry study of PDT supernatant cytotoxicity on BALB/3T3. Cells were cultured 48 hours in control cell culture (Bovine Calf Serum (BCS) at 2%, 10% and SDS 0,01%) and PDT supernatants from ten samples. Percentage of annexin-V negative and Propidium iodide (PI) negative cells is represented. Data were analyzed using one-way ANOVA (**** = p < 0.0001). (**D**) Haematoxylin and eosin-saffron (HES) staining of representative PDT alone, and re-cellularized PDT with BEAS cells. BEAS attached to perichondrium (white arrow). BEAS in conjonctive tissue (balck arow). Scale bars = 250 µm. (**E**) Calibration measures of colorimetric dosage of SDS. Range of SDS concentrations (triplicate) was used to calibrate the instrument and check the reliability of the measurements. Cytoxicity threshold is represented, as found in (A). (**F**) Adsorption of SDS by the cartridge. H0 Measure was significant. From H1 onwards, absorbance measurements were below the instrument's detection limit. (**G**) Colorimetric measurements of SDS release per 30-mg PDT samples. Black dots are the calibration points. Four PDT were tested (white dots).

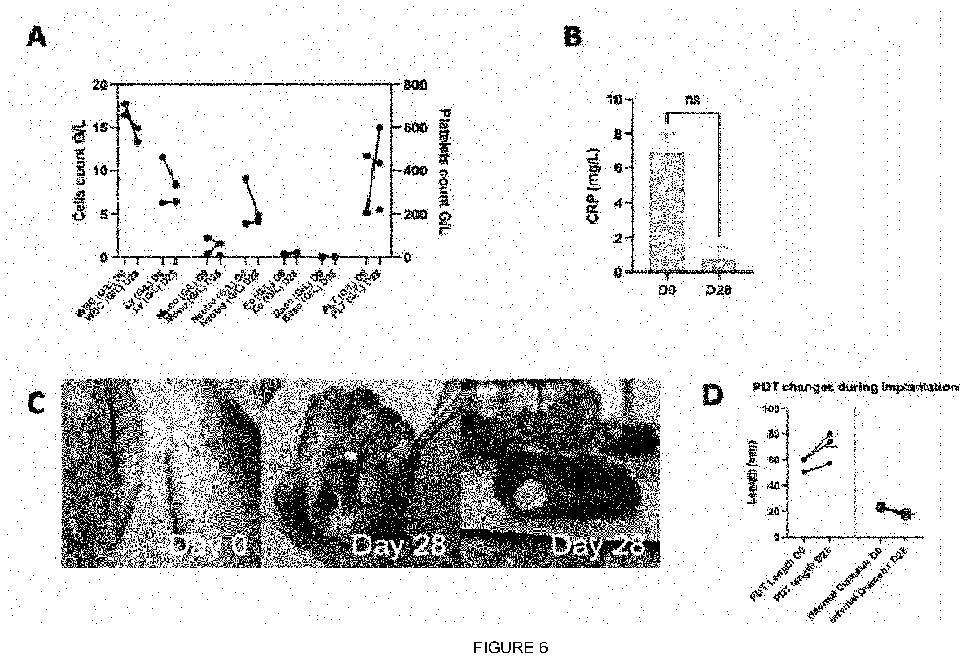

Figure 6 shows *in-vivo* maturation of PDT. (**A**) Evolution of white blood cells (WBC), lymphocytes (Ly), monocytes (Mono), granulocytes neutrophils (Neutro), Eosinophiles (eosino), basophils (baso) and platelets (PLT) during implantation. (**B**) C-Reactive Protein dosage at implantation (Day 0) and explantation (Day 28). (**C**) The macroscopic aspect of the partially decellularized trachea (PDT) is shown before implantation and after implantation. Before implantation (Day 0), two of the four tension knots are visible. After 28 days of maturation (Day 28), PDT has been explanted with surrounding tissue: top view, scar tissue is seen between PDT and sterno-cephalic muscle (white star). In the lateral view, there was no collapse and no inflammation inside the matured PDT. (**D**) Representation of changes in length and internal diameter of the trachea during implantation in a muscle. Measurements were taken at the time of implantation, then after explantation and stent removal.

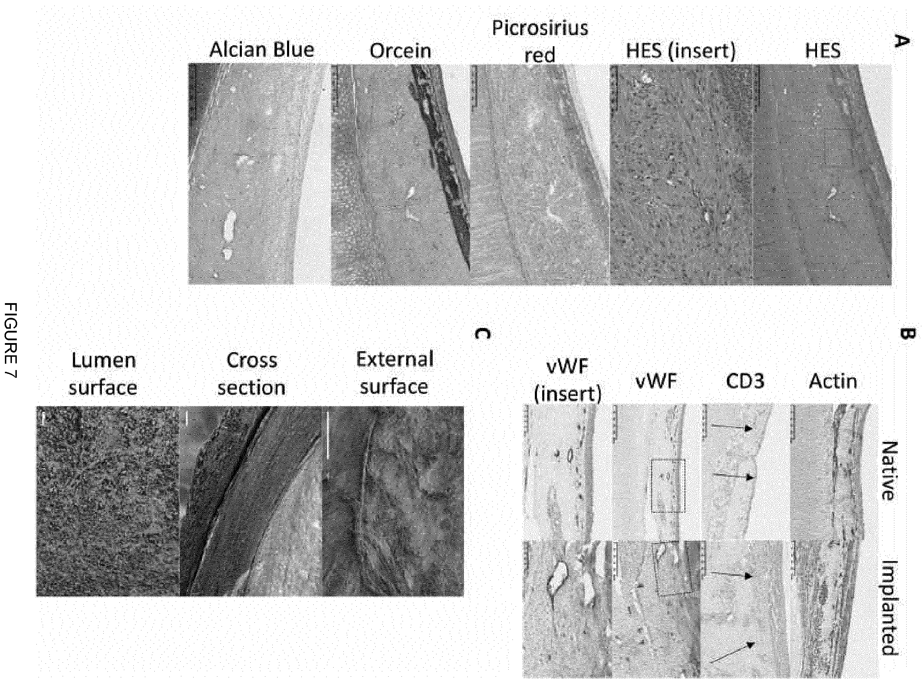

Figure 7 shows immunity and inflammation response to implantation. (**A**) Histological studies of a partially decellularized trachea (PDT) after 28 days of muscular implantation are shown using hematoxylin-eosin-saffron (HES) for the general structure, Picrosirius red (PSR) for collagen, orcein for elastic fibers and Alcian blue for glycosaminoglycan staining. Scale bar = 500 µm, except insert 100 µm. (**B**) Comparison of immunostaining between native trachea and reimplanted PDT using Actin, CD3 and Von Willebrand Factor (vWF) antibodies. CD3+ cells are shown with black arrows. Scale bar = 250 µm, except insert, scale bar = 100 µm. (**C**) Scanning electron microscopy (SEM) images of external surface, cross-section and lumen surface of matured PDT sample. Scale bar = 100 µm.

### EXAMPLES

### EXAMPLE 1 : MATERIALS AND METHODS

### 1. Tracheal Harvesting

Tracheas from Large White/Landrace pigs weighing between 50 and 70 kg were surgically removed after euthanasia. They were collected from freshly sacrificed animals in a professional slaughterhouse of a national research facility (Institut National de Recherche pour l'Agriculture, l'Alimentation et l'Environnement, Nouzilly, INRAe, France). Harvesting included the cricoid, trachea, carina and first rings of the pig bronchus and stem bronchi on each side.

### 2. Tracheal decellularization

The decellularization process consisted of four major steps: decontamination, treatment with detergent to remove the cells and their contents, rinsing and incubation with an activated charcoal cartridge to remove residual detergent and antibiotics, and treatment with DNase to remove remaining genetic material. All these steps were performed under sterile conditions in a laminar flow hood.

### 2.1 Decontamination

After resection, each trachea was rinsed, cleaned of connective tissue and placed in a 500 mL bottle which was used until the end of the process. Tracheas were decontaminated overnight under constant agitation (230 rpm, New Brunswick Innova, 42 R shaker, Eppendorf, Hamburg, Germany) in a solution of antibiotics and antimycotics: 320 mg/l gentamycin (Panpharma, Luitré, France), 600 mg/l clindamycin (Pfizer, Paris, France), 500 mg/l vancomycin (Mylan, Saint-Priest, France), and 100 mg/l amphotericin B (Bristol-Myers Squibb, Rueil-Malmaison, France) at 24°C.

### 2.2 Decellularization protocol

Solution was changed to 1% sodium dodecyl sulfate (SDS; Euromedex, France) diluted in sterile water and bottles were placed under orbital agitation (230 rpm) at 24°C (New Brunswick Innova^{®} 42R, Eppendorf France, Montesson). This process was performed in order to remove epithelial and submucosal cells. After 24 hours, tracheal segments were rinsed with saline for four 20-minute cycles at 37°C. Then, in order to remove residual SDS, an activated charcoal cartridge was used. The bottles were filled with sterile water and connected in series to the Adsorba 300C cartridge (Baxter, USA), forming a closed circuit with a peristaltic pump (27mL/min). The bottle was placed on a warming magnetic agitator Cimarec^{™} (Thermo Fisher Scientific Inc., Waltham, USA) and the solution was continuously filtered for 96h at 37°C and 270 rpm. This way, the SDS released from the decellularized matrix was adsorbed by the cellulose-coated activated charcoal (Figure 1). To remove DNA, the matrix was incubated for 3 h with DNase 40 UI/ml (Pulmozyme, Roche, Boulogne-Billancourt, France) at 37°C under constant orbital agitation (230 rpm) in New Brunswick Innova^{®}42R. The final partially decellularized trachea (PDT) was then rinsed and stored in phosphate buffered saline (PBS, Eurobio, Courtaboeuf, France) at 4°C before further evaluation.

### 2.3 Cryopreservation

When cryopreserved, PDTs were stored in a solution of Roswell Park Memorial Institute Medium (RPMI, Gibco^{®} Waltham, MA, U.S.A.) with 10% DMSO (CryoSure-DMSO, WAK-Chemie Medical GmbH, Germany) and 0.8% albumin (Albumine, OctaPharma, Boulogne Billancourt). The descent in temperature was done in two steps: a freezing at -20°C overnight, then long term preservation at -80°C.

### 3. Histological and immunostaining analysis

### 3.1 Histology

Tracheal segments were fixed in 4% formol and embedded in paraffin. Five-micrometer-thick sections were stained with hematoxylin-eosin-saffron (HES) for general structure and remaining cells, picrosirius red for collagen fibers, orcein for elastic fibers and alcian blue for glycosaminoglycans.

### 3.2 Immunostaining and immunofluorescence

The immunostaining and the immunofluorescence required deparaffinization of histological sections. Slides were rinsed with PBS, fixed with 3% paraformaldehyde diluted in PBS (15 min) then rinsed with PBS containing 0.5% BSA (Bovine Serum Albumin). They were permeabilized with 0.1% Triton X100 in PBS (4 min) for 30 minutes at room temperature, then rinsed 3 times with PBS-BSA.

**3.3 Immunostaining** was performed with antibodies directed against CD3 (clone SP7; rabbit monoclonal antibody; Epredia Netherlands, Da Breda, NL), actin (clone 1A4, mouse monoclonal antibody; Agilent Dako, Agilent Technologies France, LES ULIS) and von Willebrand factor (rabbit polyclonal antibody; Agilent Dako).

**3.4 Immunofluorescence** was performed with an association of a primary antibody and a secondary immunofluorescent antibody. The following primary antibodies were used: Collagen II (ab34712 rabbit polyclonal IgG, Abcam) and Laminin (L9393, rabbit polyclonal IgG, Merck). Histological sections were incubated with the primary antibodies for 2 hours at room temperature. After several washes in PBS-BSA, slides were incubated for 1 hour at room temperature with the appropriate secondary antibody: Anti-Rabbit A11008. Antibodies were diluted in PBS-BSA. DNA from cell nuclei was labeled with DAPI (4,6-diamino-2-phenyliindol dihydrochloride, D9542, Sigma Aldrich). The coverslips were mounted in Prolong-Gold antifade reagent (P36930, Invitrogen). Specificity controls were performed by incubating tissues with the secondary antibody alone, used as negative control. Autofluorescence analysis of histological sections was also performed. Immunofluorescent labeling observations were made under a Zeiss Axio Observer Z1 microscope equipped with an axiocam305 sCMOS camera. The lens was EC Plan neofluar x10/0.3.

### 4. Decellularization efficiency and quality control

### 4.1 Sterility

According to the standards of clinical grade human tissue banking, sterility was evaluated on samples of trachea (1) before and (2) after antibiotics and antimycotics treatment, (3) at the end of the whole decellularization process and (4) after thawing of cryopreserved PDT samples. Samples were incubated in Schaedler broth (Biomérieux SA, Craponne, France) for 10 days and then seeded into Chocolate agar + PolyViteX (Biomérieux SA) for aerobic culture, Columbia agar + 5% sheep blood (Biomérieux SA) for anaerobic culture, and Sabouraud chloramphenicol gentamicin agar (Bio-Rad Inc, Hercules, USA) for fungal culture. The bacterial media were incubated at 37°C for 8 days and the fungal medium at 30°C for 11 days. In addition, incubated Schaedler for samples (3) and (4) were seeded in aerobic and anaerobic BACT/ALERT^{®} - Flasks (Biomérieux, France) for 10 days at 37°C.

### 4.2 DNA quantification and electrophoresis on agarose gel

Native tracheal and PDT samples were collected; the mucosa was separated from the rest of the trachea containing cartilage rings. Samples of about 30 mg were cut and weighed. They were digested overnight with proteinase K and DNA was extracted using the PureLink^{™} Genomic DNA Mini Kit (Invitrogen, USA) according to the manufacturer's instruction. The extracted DNA was quantified by Nanodrop (Thermofisher) and the concentration was calculated per milligram of wet tissue mass. The size of the DNA fragments was assessed after electrophoresis in a 2% agarose gel containing SYBR^{®} safe (Thermofisher). The gels were observed with iBright1500 (Thermo Fisher Scientific). GeneRuler 1 kb (Thermo Fisher Scientific) ladder was used for size control.

### 4.3 Scanning electron microscopy

Segments of native trachea, PDT and matured PDT were fixed with Paraformaldehyde 2 %, Glutaraldehyde 2.5 %, Sodium cacodylate 0.2M pH 7.3. After washing in cacodylate buffer, segments were cut into 0.25 cm² pieces, dehydrated through graded concentration of ethanol from 30% to 100%, critical point dried (CPD300, Leica), mounted on stubs and finally sputtered with 4 nm of platinum (ACE600, Leica). Scanning Electron Microscopy was conducted using a field emission gun Scanning Electron Microscope (GeminiSEM300, Carl Zeiss) with an acceleration voltage of 2 keV under high vacuum. Secondary electrons were collected. Scan speed and line averaging were adjusted during observation.

### 4.4 Biomechanical evaluation

All biomechanical testing was performed at 23°C on a single-column, tabletop, universal testing machine (UTM) (model 5844; Instron Corp., Norwood, MA) equipped with a 2 kN load cell (Instron). Software (Merlin version 5.53.00; Instron) was used to control test execution and data collection. Four-ring specimens of native tracheas, cryopreserved native tracheas and PDT were equilibrated to room temperature prior to testing and kept hydrated in PBS for mechanical testing. They were subjected to uniaxial radial compression testing by placement between two metal plates with the membranous side down. The upper plate was attached to the UTM load arm and lower plate was attached to the fixed UTM baseplate. Each specimen was compressed at a rate of 2.5 mm/min until 90% to 100% lumen occlusion was visible, at which point the test was stopped manually. Compressive load (N) versus compressive displacement (mm) data were obtained, and the load at 50% compressive displacement was calculated. Percent displacement was defined as Dx /D0, where Dx was the distance traveled, and D0 was the starting position.

### 4.5 In-vitro biocompatibility of the PDT

### 4.5.1 Preliminary study of SDS cytotoxicity threshold

BALB/3T3 clone A31 cells (ATCC, U.S.A.) were grown in DMEM (ATCC, U.S.A.) supplemented with 2% bovine calf serum (BCS, ATCC, Manassas, VA, USA) and 1% ATB/ATM. BALB/3T3 cells were seeded at 1.5×10⁴ cells/well in a 48-well plate in a medium to which SDS had been added at a concentration ranging from 1% to 10⁻⁷%.

After 48 hours, cell viability was assessed using the LIVE/DEAD^{®} viability/cytotoxicity kit (ThermoFisher Scientific) containing calcein and ethidium, within a live cell fluorescence microscope (Incucyte, Sartorius). Positive controls were DMEM with 10% and 2% BCS. Negative control was culture medium added with 1% SDS. Images were acquired using a live microscope, Incucyte^{®} (Sartorius).

### 4.5.2 Cytotoxicity assay

This assay was designed according to European guidelines (Iso 109935-2009). BALB/3T3 clone A31 cells were grown in DMEM supplemented with 2% BCS and 1% antibiotics and antimycotics. 5-mm diameter samples of PDT were prepared with biopsy punches (Kai medical, Solingen, Germany) and incubated in 500µl of cell culture medium. After 48 hours, the supernatants were transferred on BALB/3T3 cells seeded at 1.5×10⁴ cells/well in a 48-well plate for 48 hours. Cell viability was screened as in the previous experiment using the LIVE/DEAD^{®} viability/cytotoxicity kit with live Incucyte^{®} (Sartorius).

Cell viability was also quantified by flow cytometry. At the end of the 48-hour culture, floating cells in the supernatant were collected and the remaining cells were detached by a trypsin treatment. All cells were centrifuged and suspended in 1X apoptosis buffer (Invitrogen). They were stained with 2µl/tube Annexin V-FITC and 2µl of Propidium iodide (PI) kit (Invitrogen). After 15 mins of staining at room temperature, cell viability was analyzed by flow cytometry, using an Attune-Nxt cytometer (Invitrogen, Thermo Fisher Scientific). Unstained cells were used as control for fluorescence. Viability positive controls were cells cultured in medium with 10% and with 2% BCS. Negative control was cells cultured in medium with 2% BCS containing 0.01% SDS. Annexin V-/PI- population was considered as viable.

### 4.5.3 Residual SDS quantification

The concentration of residual SDS was measured by a spectrophotometric method, based on the use of a carbocyanine dye (Stains-all) the color of which changes from intense fuchsia to yellow upon addition of SDS.

### Buffers and Reagent Solutions

Stains-all was dissolved in N, N-dimethylformamide to give a stock solution of 2.0 mg/ml. Working solutions were diluted 1:20 with HPLC water into dark amber Falcon^{™} tube and stored in the dark at - 8 °C.

### Standards preparation

SDS concentration standards were prepared by dilution of the 20% stock solution with PBS, ranging from 0.01% to 0.00025% SDS and stored at room temperature. The linearity of the curve representing absorbance *versus* concentration was validated prior to the dosage.

### SDS adsorption capacity of the Clinical-grade cartridge

The SDS adsorption capacity of the clinical-grade charcoal cartridge was validated using a 0,005% SDS solution, filtered in a closed circuit. Iterative samples were taken before starting the pump, every hour for 8 hours, then H24, H72 and H96.

### SDS released from PDT

Samples of PDT were lyophilized and stored at 4°C. Samples of 30mg were placed into 2mL tubes with 1mL of PBS. They were grinded with a Tissue Lyser (Qiagen) at 50 rpm for 15 mins and placed in an incubator for 48h at 37°C. Supernatants were collected and analyzed for the dosage of SDS.

### Spectrophotometric analysis of detergent concentration

All microtiter assays were performed in a Dulbecco PBS buffer.

PBS buffer (210 µl/well) was dispensed using a multichannel pipet. 15 µl of each standard, or sample was dispensed into the well of a 96 wells "ultra-low UV" (Corning). A multichannel pipet was then used to dispense 75 µl/well of Stains-all working solution. The microtiter plate was immediately placed in the incubator/reader and shaken for 20 s, allowed to incubate unshaken for 20 s, and then read for absorbance using a 450 nm filter with a microplate reader « Varioskan Lux » (Thermo Scientific).

### 4.6 Epithelial cell viability on PDT - Cell seeding with BEAS-2B cell line:

Human bronchial epithelial cell line BEAS-2B (Merck) was seeded and amplified in SAGM^{™} Small Airway Epithelial Cell Growth Medium BulletKit^{™} (Lonza, USA), in collagen coated BioCoat^{™} flasks (Corning, USA). Once confluent, cells were detached by trypsin treatment and were resuspended at a concentration of 4×10⁶ cells/mL.

5-mm diameter punches of 4 different PDTs were prepared. Three punches/PDTs were placed in ultra-low-fixation 24-well plates (Corning, USA). Resuspended BEAS-2B were then placed in contact with these samples. The plates were placed on an agitator (Grantbio), in a cell culture incubator at 37°C, 5% CO₂ for a week. They were then fixed for 24h in paraformaldehyde (PFA) 4%, included in paraffin and 5µm thick sections were prepared. Samples were evaluated by histology. BEAS-2B adherence and distribution was assessed by HES staining.

### 5. In vivo study in pigs

### 5.1 Animal care and ethics statement

Female hybrid Landrace, Large White and Pietrain pigs aged 4 months, weighing between 50kg to 60kg, and issued from the same breeding were used. All experiments were carried out in the same accredited animal facilities. The experimental protocol was carried out in accordance with EU Directive 2010/63/EU and institutional guidelines for animal experiments. It was approved by the scientific committee of our institution, the ethics committee for animal experimentation n°INSERM-034, and the Ministry of National Education, Higher Education and Research (agreement number: project n°2022120817007429 - V8 APAFiS # 42094).

### 5.2 Anesthesia/asepsis protocol

All procedures were performed in sterile conditions in an operating room inside research facilities of Laboratoire de Recherches Biochirurgicales de la Fondation Alain Carpentier [PARCCeINSERM], under general anesthesia after a fasting period of 24 hours. The induction was performed by an injection of 4 mg/kg of propofol and 0.5 mg/kg of morphine hydrochloride intravenously. The animals were intubated and placed under controlled ventilation. Anesthesia was achieved by inhalation of 3 - 5% isoflurane in 100% oxygen delivered at a rate 1 L/min. A perfusion of Ringer's lactate was implemented at a rate of 10 - 20 mL/kg/h. Antibiotic prophylactic therapy was performed with an intravenous injection of 15 mg/kg cefamandole.

### 5.3 In-vivo maturation of the PDT

The skin incision was a 15 cm median sagittal cervical incision. This approach enabled dissection of the sternocephalic muscle with preservation of the superior-anterior vascular pedicle and collaterals, and minimal dissection of the tracheal, thyroid and esophageal structures. Prior to implantation, the PDT was stented and stretched with a clinical-grade silicone stent (Rutter supra-stomal stent, Boston Medical Products, Shrewsbury, MA, USA). The stent was occluded on both sides. The PDT was then tensioned over the stent with two Ethibond 2/0 sutures (Ethicon, Johnson&Johnson France, 92787 Issy-les-Moulineaux) at each end. The objectives of stenting were to avoid PDT retraction, proliferation of endoluminal fibroblasts and fluid sequestration that favors infection. The tensioning was intended to optimize the contact surface of the inter-annular spaces with the recipient's muscle tissue, to promote neovascularization. The stented PDT was placed within the sterno-cephalic compartment during 30 days. This compartment was sutured to itself using Vicryl 2/0 (Ethicon, Johnson&Johnson France, 92787 Issy-les-Moulineaux). The white line was sutured with Vicryl 2/0. The skin was sutured in 3 plans with Vicryl 2/0. Antibiotic spray was applied to the skin scar. The scar was left open air for reasons of poor dressing tolerance and clinical monitoring.

### 5.4 Blood cell count and CRP quantification

Blood samples were taken during each general anesthesia: before implantation of the PDT (Day 0) and before explantation of the PDT (Day 30). K3-EDTA tubes (BD Vacutainer, ref 368270, Becton Diskinson France, Rungis) were used for complete blood count, and tubes containing a separating gel and coagulation activator (BD Vacutainer, SST^{™} II Advance, ref 367953, Becton Dickinson France, Rungis) were used to prepare serum samples for C-reactive protein analysis. Complete blood counts were performed on EDTA whole by XN analyzers (Sysmex) and reviewed by a senior hematologist familiar with porcine blood cells. Serum samples were prepared by centrifugation of the tubes at 3000 rpm. All samples were frozen at -80°C before analysis.

C reactive protein (CRP) was quantified by ELISA, using the Porcine C-Reactive Protein/CRP DuoSet ELISA kit (R&D systems), according to the manufacturer's instructions. Absorbance at 450 nm was measured using a VarioskanLux plate reader (Thermo scientific).

### 6. Statistics

Commercial computer statistical software (GraphPad Software Inc., USA) was used for statistical analysis. A p-value of less than 0.05 was considered to be statistically significant. Descriptive statistics are presented as column bars with mean and standard deviation. Simple linear regression was used for the validation of colorimetric standard curve. For comparison of non-parametric distribution Mann-Whitney test was used (DNA concentration). For comparison of mean, t-test (CRP) or one-way ANOVA (cell viability) have been used.

### EXAMPLE 2: RESULTS

### 1. Partial decellularization protocol is effective

### 1.1 Sterility is obtained after decontamination

All the tested tracheas were positive for bacterial and fungal growth before decontamination. The decontamination step with ATB/ATM removed all bacterial and fungal contamination from all the samples. After the decellularization process, every sample was negative for bacterial and fungal presence (Table 1). After cryopreservation and thawing, no contamination was reported, neither bacterial nor fungal.

**Table 1: microbiological controls at different stages of the process**

| Samples | Before decontamination with antibiotics | After 24hrs of decontamination with antibiotics | After full decellularization | After decellularization, cryopreservation and thawing |
|---|---|---|---|---|
| #1 | Positive | Negative | Negative | Negative |
| #2 | Positive | Negative | Negative | Negative |
| #3 | Positive | Negative | Negative | Negative |
| #4 | Positive | Negative | Negative | Negative |

### 1.2 Histology

To assess the efficacy of the decellularization protocol, histological examinations were performed on native tracheas, fresh and cryopreserved PDTs. HES staining showed that partial decellularization removed all cells and nuclei from the epithelium, the mucosa, the submucosal glands, the muscle, the vessels, but not from the tracheal cartilage as expected (Figure 2). Picrosirius red (PSR) staining demonstrated the preservation of collagen within tracheal scaffold after decellularization and cryopreservation. Orcein staining demonstrated the retention of elastic fibers in the PDT and cryopreserved PDT. Blue alcian staining demonstrated the retention of glycosaminoglycans (GAGs) into the cartilage and the sub-mucosa after tissue engineering and cryopreservation (Figure 2).

A dense DNA staining by DAPI was observed in the epithelium and chondrocyte nuclei in the native trachea (Figure 3). In PDT, DNA staining by DAPI was only visible within the cartilage. By immunofluorescence, contrarily to the native trachea, laminin was less organized in the basement membrane of PDT. Type II collagen staining was present at the edge of mucosa but appeared more disorganized within PDT.

### 1.3 DNA quantification

Within the mucosa, the DNA content of PDT compared to native trachea was significantly reduced from 344.8 ± 244.8 ng/mg to 33.42 ± 26.7 ng/mg, corresponding to a 90,4% reduction (Figure 4A). These values were below 50 ng/mg and were therefore in accordance with Badylak's values for tissue decellularization standards(Badylak, Taylor and Uygun, 2011).

### 1.4 DNA electrophoresis on agarose gel

Mucosa samples from matched native tracheas and PDT were assessed. DNA fragment size was evaluated by migration on agarose gel. Size of DNA fragment was estimated by comparison with the DNA ladder on the left-hand side. In the native trachea, DNA fragment size exceeded 1000 bp. In the PDT mucosa, no DNA fragments were visible (Figure 4B). This result is also in compliance with Badylak's decellularization standards(Badylak, Taylor and Uygun, 2011).

### 1.5 Scanning electron microscopy

Scanning electron microscopy (Figure 4C) of native trachea showed the presence of a lining of ciliated epithelial cells and goblet cells within the lumen. As for the cartilage, it was composed of a dense ECM, including chondrocytes. After decellularization, all epithelial cells were removed but chondrocytes were still present. As for the ECM, despite some disorganization of the fibers on the outer side of the PDT, no damage was visible in the cross-section of the cartilage, suggesting that the decellularization process does not negatively impact the cartilage morphology.

### 1.6 Biomechanical properties

Cryopreserved and decellularized tracheal segments are less resistant to pressure than native trachea segments (Figure 4D) with a 51.7% decrease from 1.83N (Native, n=34) to 0.9N (cryopreserved trachea, n=8) and 0.95N (PDT, n=9) Nevertheless, we did not observe any spontaneous collapse, and the measured values remained within normal limits.

### 2. PDT was not cytotoxic in-vitro

### 2.1 Preliminary study of SDS cytotoxicity threshold

### 2.1.1 Preliminary study of SDS cytotoxicity thresholds

Positive controls, corresponding to cells cultured in medium containing 10% or 2% BCS, showed a high viability (Figure 5A). The range of SDS dilutions on Balb/3T3 cells showed that they were alive from 0.006% SDS-concentration and below, corresponding to 57ng/mL. At slightly higher concentrations (0,007%), cell death was reproductible. This concentration of 0.006% was subsequently adopted as the cytotoxicity threshold for SDS.

### 2.1.2Cytotoxicity assay on Balb/3T3 cells

In live microscopy, the PDT supernatant was still toxic on Balb/3T3 after 48 hours of filtration on the charcoal cartridge, showing that SDS released from the matrix reached cytotoxic concentrations. After 96 hours of detoxification, the PDT supernatant was not cytotoxic anymore (Figure 5B). This result was confirmed by flow cytometry: at 96h, the percentage of viable Balb/3T3 cells (Annexin negative/ PI negative) cultured in the presence of PDT supernatant was not different than the control conditions, with a viability of 83,2% and 91,7% respectively (Figure 5C). These results demonstrated the absence of cytotoxicity induced by the PDT after the partial decellularization process.

### 2.1.3 BEAS-2B epithelial cell adhesion and viability on PDT

Cell seeding with epithelial BEAS-2B cell line showed that these cells could adhere to the PDT, cartilage or connective tissue, and proliferate (Figure 5D). However, cell repartition was not homogeneous. Comparison with the PDT patch, without BEAS cells, confirms the absence of infiltration and colonization inside the cartilage. These results confirm the biocompatibility of the PDT *in-vitro,* by a direct method.

### 2.1.4 Residual SDS quantification

The activated charcoal cartridge was effective at capturing SDS diluted in water as there was no detectable SDS after one hour of filtration (Figure 5E, 5F). Nevertheless, this rapid detoxification time corresponded to free SDS in a solution. Therefore, in order to evaluate the residual SDS trapped in PDT, we performed SDS quantification on PDT, after 96 hours of detoxification. No SDS could be detected in the supernatant of the samples, showing an efficient removal of this detergent from tissues as well (Figure 5G).

### 3. PDT was biocompatible in-vivo

PDT were implanted in 3 pigs for a 28 days period. The pigs were monitored daily during the maturation period. No episodes of fever, general or local infection were reported. The pigs were in good health and showed no particular discomfort from the presence of the PDT or the scar.

### 3.1 Blood analysis

Complete blood cell counts before implantation (Day 0) and before explantation (Day 28) were compared (Figure 6A). On Day 28, there was no hyperleukocytosis or anemia. One pig had an isolated increase in platelets, with no clinical explanation, except a centimetric sub-cutaneous abcess. C-Reactive protein decreased during the implantation period, with no clinical evidence. The concentrations detected (D0: 6.9mG/L, D28: 0.7mG/L) remained within a physiological range (Figure 6B).

### 3.2 Macroscopy and biomechanics

Macroscopically, on excision, PDT were found within a typical fibrous gangue of a foreign-body reaction. There were no local signs of inflammation or infection, except for one pig who had a 1-cm subcutaneous abscess on a suture, not seen during clinical follow-up, with no extension to the implantation zone. After stent removal, the PDT was of satisfactory caliber, the cartilage rings were intact, there was no necrotic area. We performed a rough test of the biomechanical properties: to the touch, they appeared to have properties similar to those of native tracheas. They did not collapse and maintained their caliber over time (Figure 6C).

The length of the matured PDT was increased (mean 23,5%, 14 - 33), due to the tension imposed during the maturation phase (Figure 6D). The purpose of this tension was to better expose the interannular ligaments to facilitate vascularization. The diameter, on the other hand, was reduced (24,5%, 17 - 33). During maturation, the PDTs retracted around the 15-mm endoluminal stent.

### 3.3 Histology

HES staining showed that the overall architecture of the PDT was preserved. On all sections analyzed, the cartilaginous ECM was intact. There was neither cartilage, nor contact muscle lysis. The inner surface of the trachea was the site of a large fibrinous neo-tissue formation in the process of reorganization. Numerous myofibroblasts had colonized the PDT (Figure 7A, HES and HES insert). This neoformed tissue was also seen in the external tunica of the trachea where it distended the intercartilaginous spaces. Numerous neovessels were seen visible. Elastic fibers were still present in the submucosal site, as were glycosaminoglycans (Figure 7A).

In immunostaining, there was no massive lymphocyte infiltration, few CD3⁺ cells were visible in the submucosal space. Neovascularization was confirmed by a large amount of endothelial cells, labeled with the vWF antibody (Figure 7B)

### 3.4 Scanning electron microscopy (SEM)

The structure of the trachea was preserved after a 28-day maturation period in the muscle (Figure 7C). On cross sections, the cartilaginous matrix seemed intact and well conserved, as did the epithelial basal lamina . Signs of bio-integration were evident: a vascular network was observed on the outer surface of the trachea, and numerous cells colonized the inner surface. These data confirmed the histological aspect.

### List of references

1. Grillo et al., Thorax, 28(6): 667-679, 1973
2. Fabre et al., the Annals of Thoracic Surgery, 96(4) : 1146-1155, 2013
3. Kolb et al., New England Journal of Medicine, 378(14) : 1355-1357, 2018
4. Kubo et al., Microsurgery, https://doi.org/10.1002/micr.30365, 2018
5. Thomet et al., Annals of PlasticSurgery, https://doi.org/10.1097/SAP.0000000000001399, 2018
6. Parshin et al., Khirurgiya. Zhurnal im. N.I. Pirogova, https://doi.org/10.17116/hirurgia201811111, 2018
7. Genden et al., American Journal of Transplantation, 21(10): 3421-3427, 2021
8. Greaney and Niklason, Tissue Engineering Part B: Reviews, 27(4): 341-352, 2021
9. Kaye et al., International Journal of Pediatric otorhinolaryngology, 117: 175-178, 2019
10. Park et al., Scientific Reports, 9(1) : 2103, 2019
11. Wurtz et al., N Engl J Med., 355: 1938-1940, 2006.
12. Wurtz et al., The Journal of Thoracic and Cardiovascular Surgery, 140(2) : 387-393, 2010
13. Martinod et al., JAMA, 319(21): 2212, 2018
14. Elliott et al., The Lancet, 380(9846) : 994-1000, 2012
15. Elliott et al., STEM CELLS Translational Medicine, 6(6) : 1458-1464, 2017
16. Culme-Seymour et al., Tissue Engineering Part A, 22(3-4) : 208-213, 2016
17. Liu et al., The Journal of Thoracic and Cardiovascular Surgery, 120(1): 108-114, 2000b
18. Liu et al., ASAIO Journal, 46(5) : 536-539, 2000a
19. Liu et al., The Annals of Thoracic Surgery, 72(4) : 1190-1194, 2001
20. Delaere et al., New England Journal of Medicine, 362(2): 138-145, 2010
21. Aoki et al., Scientific Reports, 9(1) : 12034, 2019
22. Liu et al., Journal of Tissue Engineering, 12 : 204173142110174, 2021
23. Liu et al., ASAIO Journal, 48: 21-25, 2002
24. Lu et al., European Journal of Cardio-Thoracic Surgery, 53: 672-679, 2017.

## Claims

1. Partial tracheal decellularization tissue engineering method comprising the following steps:
a) partial decellularisation of a trachea previously sampled from a mammal with a detergent;
b) rinsing the partially decellularized trachea recovered from step a) at least once with physiological saline solution to remove the detergent;
c) contacting the partially decellularized trachea recovered from step b) with active charcoal to adsorb residual detergent;
d) contacting the partially decellularized trachea recovered from step c) with a deoxyribonulease to remove residual DNA;
e) rinsing the partially decellularized trachea recovered from step d) at least once with physiological saline solution to remove the deoxyribonuclease;
f) optionnaly, cryopreservation of the partially decellularized trachea recovered from step e).

2. Method according to claim 1, wherein the sampled trachea of step a) is deffated and decontamined before decellularization.

3. Method according to claim 1 or 2, wherein the mammal from which the trachea was previously sampled is selected from a mammal of more than 3 kg.

4. Method according to any of claims 1 to 3, wherein the mammal is selected from pig, human, dog, sheep, goat, cow, non-human genetically modified mammals and humanized mammals.

5. Method according to any of claims 1 to 4, wherein step a) is carried out between 12 to 36 hours, at room temperature.

6. Method according to any of claims 1 to 5, wherein the detergent of step a) is selected from Sodium Dodecyl Sulfate (SDS), Triton X-100, Tween-20, sodium deoxycholate.

7. Method according to claim 6, wherein the detergent is SDS 1%.

8. Method according to any of claims 1 to 7, wherein step c) is carried out during 96 hours, at room temperature.

9. Method according to any of claims 1 to 8, wherein step c) and e) are carried out three times with stirring.

10. Method according to any of claims 1 to 9, wherein the partially decellularized trachea in step f) is placed at -20°C in RPMI medium during 12 hours then stocked at -80°C.

11. Partially decellularized trachea obtained by a method according to any of claims 3 to 10, wherein the tunica interna of the trachea is completely decellularized.

12. Partially decellularized trachea obtained by a method according to any of claims 1 to 10 or partially decellularized trachea according to claim 11, for use as a medicament.

13. Partially decellularized trachea for use according to claim 12, wherein the medicament is a paediatric medicament.

14. Partially decellularized trachea obtained by a method according to any of claims 1 to 10, for use in the prevention or treatment of acquired or congenital stenosis of the trachea such as congenital tracheal stenosis, large tracheoesphageal fistula, tracheal agenesis, tracheal tumor, laryngotrachealoesphageal cleft, tracheomalacia.
